# EUROPÄISCHE PATENTSCHRIFT

(11) **EP 1 888 148 B1**
(45) Veröffentlichungstag und Bekanntmachung des Hinweises auf die Patenterteilung: **10.10.2012**
(21) Anmeldenummer: 06721939.4
(22) Anmeldetag: 28.04.2006
(51) Int. Cl.: A61M 5/315, A61M 5/24

(54) **DOSIERVORRICHTUNG FÜR EINE INJEKTIONSVORRICHTUNG**
DOSING DEVICE FOR AN INJECTION DEVICE
DISPOSITIF DE DOSAGE POUR SYSTEME DE SERINGUE

(30) Priorität: 24.05.2005 DE 102005023824
(43) Veröffentlichungstag der Anmeldung: 20.02.2008
(73) Patentinhaber: TecPharma Licensing AG, 3401 Burgdorf (CH)
(72) Erfinder: BURREN, Stefan, CH-3047 Bremgarten (CH); MOSER, Ulrich, CH-3412 Heimiswil (CH); SCHRUL, Christian, CH-3400 Burgdorf (CH)
(86) Internationale Anmeldenummer: PCT/CH2006/000237
(87) Internationale Veröffentlichungsnummer: WO 2006/125328

(56) Entgegenhaltungen:
- EP-A- 0 937 471
- WO-A-2004/007001
- US-A- 3 128 765
- US-A- 4 583 978
- US-A- 5 308 340

## Beschreibung

Die vorliegende Erfindung bezieht sich auf eine Dosiervorrichtung für eine Injektionsvorrichtung, insbesondere auf eine Dosiervorrichtung, mit welcher eine Dosis oder Menge einer aus- oder mit Hilfe der Injektionsvorrichtung abzugebenden Substanz eingestellt werden kann.

Aus der DE 202 09 051 U1 ist ein Injektionsgerät mit endpositionsblockiertem Dosiseinstellglied bekannt, wobei das Injektionsgerät ein Dosiseinstellglied aufweist, das für die Auswahl der Produktdosis relativ zu dem Gehäuse eine rotatorische Bewegung in eine erste Drehrichtung bis in eine Endposition und in eine entgegengesetzte Drehrichtung ausführen kann und das mit einer Fördereinrichtung, mit welcher eine ausgewählte Produktdosis aus einem Reservoir ausschüttbar ist, so gekoppelt ist, dass die Fördereinrichtung bei einer Betätigung die mittels des Dosiseinstellgliedes ausgewählte Produktedosis ausschüttet. Eine Verdrehsicherungseinrichtung verhindert eine rotatorische Bewegung des Dosiseinstellgliedes in die erste Drehrichtung über eine Endposition hinaus.

Die EP 0 828 527 B1 offenbart ein Injektionsgerät mit einer längsverschiebbaren Vorschubhülse, wobei ein Dosisaufdruck zum Ablesen einer zu injizierenden Dosis vorgesehen ist und ein Mechanismus vorgesehen ist, der ein Laden des Injektionsgeräts durch Herausziehen einer Schubstange dann verhindert, wenn der Vorrat einer Ampulle vollständig aufgebraucht ist, wobei die Dosiervorrichtung eine tatsächlich mögliche verabreichbare Dosis an einer im Bereich eines oberen Endes der Vorschubhülse angebrachten Dosisskala ablesbar ist.

Aus der deutschen Patentanmeldung Nr. 10 2005 001 159.4 ist eine Dosiervorrichtung für eine Injektionsvorrichtung mit Übersetzung bekannt, mit welcher insbesondere eine kleine Dosismenge exakt eingestellt und ausgeschüttet werden kann.

Die WO 2004/078239 A1 offenbart eine Medikamentenverabreichungsvorrichtung mit einem Gehäuse mit Innengewinde, einer Dosiswählhülse mit einem Gewinde, welches in das Innengewinde des Gehäuses eingreift, einer Drehhülse, welche lösbar mit der Dosiswählhülse verbunden ist, und einer Kupplung, welche zwischen der Dosiswählhülse und der Drehhülse angeordnet ist, wobei sich beide Hülsen in Bezug auf das Gehäuse drehen können, wenn die Dosiswählhülse und die Drehhülse gekoppelt sind. Wenn die Dosiswählhülse und die Drehhülse, entkoppelt sind, wird eine Rotation der Dosiswählhülse in Bezug auf das Gehäuse ermöglicht, während eine Drehung der Drehhülse in Bezug auf das Gehäuse gesperrt ist, wodurch eine axiale Bewegung der Drehhülse ermöglicht wird, so dass eine Kraft in die longitudinale Richtung zu dem proximalen Ende der Medikamentenverabreichungsvorrichtung übertragen wird.

Eine weitere aus dem Stand der Technik bekannte Dosiervorrichtung für eine Injectionsvorrichtung ist aus dem Dokument EP-A-0 937 471 bekannt. Der Oberbegriff des Anspruchs 1 ist auf diesem Dokument basiert.

Es ist eine Aufgabe der vorliegenden Erfindung, eine Dosiervorrichtung zur Einstellung und/oder Abgabe einer aus einer Injektionsvorrichtung abzugebenden Dosis, sowie eine Injektionsvorrichtung mit einer solchen Dosiervorrichtung und Verfahren vorzuschlagen, mit welchen eine sichere Einstellung der Dosis ermöglicht wird und das Risiko einer Fehlbedienung bei der Einstellung und/oder Abgabe der Dosis verringert wird.

Diese Aufgabe wird durch die Gegenstände der unabhängigen Ansprüche gelöst. Vorteilhafte Ausführungsformen ergeben sich aus den Gegenständen der abhängigen Ansprüche.

Gemäss einem Aspekt bezieht sich die Erfindung auf eine Dosiervorrichtung zur Einstellung einer aus einer Injektionsvorrichtung abzugebenden Dosis einer zum Beispiel in der Injektionsvorrichtung oder einer Ampulle enthaltenen zum Beispiel medizinischen Substanz, wie zum Beispiel Insulin oder Hormone, wobei die Dosiervorrichtung an einer Injektionsvorrichtung oder als Teil davon vorgesehen sein kann und eine z.B. am hinteren Ende der Injektionsvorrichtung vorgesehene und z.B. um die Längsachse der Injektionsvorrichtung drehbare Einstellhülse aufweist, welche in Umfangsrichtung eine oder mehrere Markierungen trägt, welche z.B. die Menge einer bei der jeweiligen Einstellposition aus der Injektionsvorrichtung abzugebenden Substanz oder die Menge eines bei Abgabe einer Dosis der Substanz in der Substanz enthaltenen Wirkstoffes angeben können. Mittels einer Drehung der Einstellhülse kann ein Benutzer z.B. durch Drehen einer auf der Einstellhülse vorgesehenen Markierung bis zu einer z.B. durch einen Pfeil auf der Injektionsvorrichtung vorgegebenen Stelle einstellen, welche Substanz - oder Wirkstoffmenge aus der Injektionsvorrichtung in einem nachfolgenden Abgabevorgang abgegeben werden soll. Die Dosiervorrichtung weist weiter eine Drehhülse auf, die aus der Dosiervorrichtung oder der Injektionsvorrichtung zur Vorbereitung einer Dosisabgabe bevorzugt relativ zu einem Gehäuse der Injektionsvorrichtung oder relativ zur Einstellhülse herausgedreht werden kann, wobei auf der Drehhülse und bevorzugt umlaufend auf der Aussenseite davon in Umfangsrichtung eine oder mehrere Markierungen angebracht sind, welche bevorzugt zum Beispiel vom Zahlenwert her identisch mit den auf der Einstellhülse angebrachten Markierungen sind oder diesen zugeordnet werden können. Erfindungsgemäss ist die Drehhülse so mit der Einstellhülse z.B. unmittelbar über einen Gewindeeingriff oder mittelbar über ein Zwischenelement gekoppelt, dass die Drehhülse im vollständig oder maximal herausgezogenen Zustand, wobei sich die maximale Herauszieh- bzw. Herausdrehbewegung der Drehhülse aus der Einstell- oder Drehposition der Einstellhülse ergibt, so relativ zur Einstellhülse positioniert, dass eine auf der Drehhülse vorgesehene Markierung nicht in Umfangsrichtung relativ zur zugeordneten Markierung der Einstellhülse versetzt ist, also in einer axialen Verlängerungen der Markierung auf der Drehhülse liegt, so dass z.B. Dosisbalken einer Dosisanzeige auf der Drehhülse im vollständig ausgezogenen Zustand der Drehhülse bei jeder Dosiseinstellung mittels der Einstellhülse in einer axialen Linie mit der entsprechenden Dosisanzeige oder Markierung der Einstellhülse liegen, wodurch die Dosis gleichzeitig an einer Seite des Pens eingestellt und das richtige Aufziehen der Dosis an der gleichen Seite des Pens einfach dadurch überprüft werden kann, dass die auf der Einstellhülse und der Drehhülse vorgesehenen zugeordneten Markierungen nebeneinander liegen. Ist dies nicht der Fall, kann ein Benutzer einfach erkennen, dass die Eingestellte Dosis nicht aufgezogen wurde und somit nicht abgegeben werden kann.

Die Einstellhülse kann mit der Drehhülse z.B. über einen Gewindeeingriff gekoppelt sein, wobei die Drehhülse z.B. koaxial in der Einstellhülse geführt werden kann, welche beispielsweise ein Innengewinde aufweist, in welches ein Aussengewinde der Drehhülse eingreift. Ebenso können Einstellhülse und Drehhülse auch entkoppelt sein, d.h. die Einstellhülse ist zum Beispiel drehbar auf der Injektionsvorrichtung gelagert, mit welcher die Drehhülse zum Beispiel in Gewindeeingriff steht. Dabei kann an der Einstellhülse ein Anschlagelement, wie z.B. ein Steg oder eine Anschlagleiste, vorgesehen sein, die z.B. in axialer Richtung, also etwa parallel zur Mittelachse der Injektionsvorrichtung zur Ermöglichung einer 360°-Drehung der Drehhülse, oder z.B. auf der Innenseite der Einstellhülse umlaufend z.B. in Form eines Gewindes oder Gewindeabschnittes zur Ermöglichung einer bis zu 720°-Drehung der Drehhülse vorgesehen ist. Auf der Drehhülse kann ein Gegenanschlag vorgesehen sein, wie z.B. ein auf der Aussenseite der Drehhülse vorstehendes Anschlagelement, um so eine radiale Anschlagsbegrenzung zu realisieren, d.h. die Drehhülse kann in der Einstellhülse soweit gedreht werden, bis das Gegenanschlagelement der Drehhülse an dem Anschlag der Einstellhülse anliegt und so eine weitere Drehung der Drehhülse relativ zur Einstellhülse verhindert, wodurch ein Aufziehen der Injektionsvorrichtung vor einer Dosisabgabe bis zu einer durch eine Drehposition der Einstellhülse festgelegten Position ermöglicht wird. Das Anschlagelement der Drehhülse kann die maximale Einstelldrehung der Einstellhülse auf z.B. etwa 360° oder 720° begrenzen. Ebenso kann ein axiales oder wendel- oder schraubenförmiges Anschlagelement auf der Drehhülse vorgesehen sein, welches mit einem oder mehreren Gegenanschlagelementen der Einstellhülse zusammenwirkt.

Die Markierung oder Markierungen auf der Einstellhülse können umlaufend z.B. ohne axialen Versatz oder z.B. alternierend mit einem wechselnden axialen Versatz vorgesehen sein, so dass z.B. die Markierungen 0.2, 0.4, 0.6 .... radial umlaufend um die Einstellhülse vorgesehen sind und axial versetzt dazu die dazwischen liegenden Markierungen 0.1, 0.3, 0.5 .... vorgesehen sein können. Auf der Drehhülse können die Markierungen ebenso wie auf der Einstellhülse vorgesehen sein oder eine Stufung oder Staffelung aufweisen, d.h., dass z.B. Markierungen, welche einen höheren Wert angeben, z.B. neben dem Versatz in Umfangsrichtung auch in distale oder proximale Richtung versetzt sein können, so dass z.B. bei einem Herausdrehen der Drehhülse aus der Einstellhülse immer der der jeweiligen eingestellten bzw. aufgezogenen Menge zugeordnete Markierungswert vollständig ablesbar ist, wenn die Einstellhülse soweit herausgedreht wurde, dass die dieser Markierung zugeordnete Dosis bei einem Eindrücken bzw. Eindrehen oder Einschieben der Drehhülse abgegeben werden kann. Markierungen, welche einem grösseren Wert zugeordnet sind, können dabei noch nicht vollständig abgelesen werden.

Anhand der auf der Drehhülse vorgesehenen Markierungen kann abgelesen werden, ob eine mittels der Einstellhülse eingestellte Dosis richtig aufgezogen wurde oder, falls eine nachfolgend beschriebene Begrenzungsmechanik, wie z.B. ein Sperrelement, in der Dosiervorrichtung enthalten ist, ob die mit der Einstellhülse eingestellte Dosis noch in der Injektionsvorrichtung oder einer Ampulle darin enthalten ist und abgegeben werden kann, so dass ein Benutzer z.B. feststellen kann, dass eine zur Verabreichung eingestellte Dosis noch oder nicht mehr aus der Injektionsvorrichtung abgegeben werden kann.

Weiterhin bezieht sich die Erfindung auf ein Verfahren zum Vorbereiten der Abgabe einer aus einer Injektionsvorrichtung abzugebenden Dosis einer Substanz, wobei mit einem Einstellelement, insbesondere einer Einstellhülse, die Menge der abzugebenden Dosis durch Drehen des Einstellelements eingestellt wird und mit einem Aufziehelement, insbesondere einer Drehhülse, die Abgabe der Substanz durch Herausdrehen des Aufziehelements vorbereitet wird, wobei aufgrund einer mechanischen Kopplung zwischen Einstellelement und Aufziehelement das Aufziehelement nach dem Einstellen der Dosis so weit bis zu einer Begrenzung herausgedreht werden kann, bis bei der Begrenzung eine auf dem Aufziehelement aufgebrachte Markierung, welche eine Dosisangabe definiert, in axialer Verlängerung zu einer zugeordneten eingestellten Dosismarkierung auf dem Einstellelement ist, sowie ein Verfahren zum Vorbereiten der Abgabe einer aus einer Injektionsvorrichtung abzugebenden Dosis einer Substanz, wobei mit einem Einstellelement, insbesondere einer Einstellhülse, die Menge der abzugebenden Dosis durch Drehen des Einstellelements eingestellt wird und mit einem Aufziehelement, insbesondere einer Drehhülse, die Abgabe der Substanz durch Herausdrehen des Aufziehelements vorbereitet wird, wobei ein bei jedem Aufziehvorgang in Richtung auf eine Endposition, insbesondere einen Endanschlag, bewegtes Sperrelement die Herausdrehbewegung des Aufziehelements begrenzt, wenn die zur Verfügung stehende Menge der abzugebenden Substanz kleiner als die mit dem Einstellelement eingestellte Menge der abzugebenden Dosis ist.

Gemäss einem weiteren Aspekt bezieht sich die Erfindung auf eine Dosiervorrichtung zur Einstellung einer aus einer Injektionsvorrichtung abzugebenden Dosis mit einer Einstellhülse, welche relativ zur Dosiervorrichtung oder relativ zu einer damit verbundenen oder gekoppelten Injektionsvorrichtung verschoben, z.B. verdreht, werden kann, um eine Menge oder Dosis einer aus der Injektionsvorrichtung abzugebenden Substanz einzustellen. Eine Drehhülse ist mit der Einstellhülse gekoppelt und kann zum Vorbereiten einer Dosisabgabe aus der Dosiervorrichtung oder Injektionsvorrichtung herausgedreht oder herausgeschraubt werden, um die Injektionsvorrichtung aufzuziehen, d.h. die Abgabe einer Substanz vorzubereiten, welche durch das Wiedereindrehen oder Einschieben der Drehhülse in die Dosiervorrichtung bzw. Injektionsvorrichtung abgegeben wird. Erfindungsgemäss ist an der Einstellhülse oder mit dieser gekoppelt ein Drehbegrenzungselement, wie z.B. ein in axialer Richtung oder wendelförmig verlaufender als Steg oder als Leiste ausgebildeter Radialanschlag vorgesehen, dessen Drehposition durch eine Einstell- oder Drehbewegung der Einstellhülse z.B. relativ zur Injektionsvorrichtung verändert werden kann. An der Drehhülse oder mit der Drehhülse gekoppelt ist ein Gegenanschlag vorgesehen, welcher bewirkt, dass die Drehhülse nur soweit aus der Einstellhülse bzw. Injektionsvorrichtung herausgedreht werden kann, bis eine Begrenzung der Drehbewegung der Drehhülse durch einen Kontakt des Anschlages der Einstellhülse mit dem Gegenanschlag der Drehhülse bewirkt wird. Somit kann je nach Ausbildung der Anschlag- und Gegenanschlagelemente ein maximaler Drehwinkel für die Drehhülse zur Vorbereitung der Abgabe einer Substanz von z.B. bei Verwendung eines einen vollen Umlauf ermöglichenden zum Beispiel axial verlaufenden Au-βenelements 360° oder z.B. 720° bei Verwendung eines zwei Umläufe ermöglichendes Anschlagelements festgelegt werden, wobei erfindungsgemäss die Einstellung einer Dosis mittels der Einstellhülse unabhängig von einer Ausdrehbewegung der Drehhülse ist, d.h. eine einmal mittels der Einstellhülse festgelegte Dosis kann ohne weiteren Einstellvorgang wiederholt aus der Injektionsvorrichtung abgegeben werden, wenn die Drehhülse immer bis zum Anschlag herausgedreht und anschliessend zur Abgabe der Substanz wieder eingedreht oder eingeschoben wird. Die Dosiseinstellung ist somit vom Aufziehen der Injektionsvorrichtung entkoppelt und kann z.B. von einem Arzt vorgenommen werden, welcher z.B. die Einstellhülse mit einem optional vorgesehenen Verriegelungselement in der Einstellposition relativ zur Injektionsvorrichtung verriegeln kann, so dass es nicht mehr möglich ist, dass ein Benutzer eine zu hohe Dosis aus der Injektionsvorrichtung abgibt. Ein Benutzer kann einfach durch ein Herausdrehen der Drehhülse bis zu einer mittels der Einstellhülse voreingestellten radialen Anschlagsposition die Injektionsvorrichtung immer richtig aufzieht. Somit kann ein "dose-memory" realisiert werden, wobei keine eigene Kopplungsbewegung zum Ein- und Auskoppeln von Einstell- und Drehhülse erforderlich ist.

Der Anschlag kann als Radialanschlag auf der Innenseite der Einstellhülse z.B. als ein in axialer Richtung oder auch spiralförmig an der Innenseite umlaufender Steg vorgesehen sein, dessen Drehposition durch eine Drehung der Einstellhülse relativ zur Injektionsvorrichtung verändert werden kann. Der Gegenanschlag kann als von der Drehhülse radial vorstehender Steg oder Nocken ausgebildet sein, welcher soweit von der Aussenseite der Drehhülse vorsteht, dass bei einer Drehung der z.B. in einem Gewinde der Injektionsvorrichtung geführten Drehhülse der vorstehende Nocken nach einem vorgegebenen Drehwinkel an dem Anschlag der Einstellhülse anliegt und somit ein Weiterdrehen der Drehhülse verhindert.

Die Einstellhülse kann, wie oben erwähnt, ein Sperr- oder Feststellelement aufweisen, mit welcher die Einstellposition der Einstellhülse z.B. relativ zur Injektionsvorrichtung festgestellt oder arretiert werden kann. Ebenso ist es möglich, dass die Einstellhülse nur relativ zur Injektionsvorrichtung bzw. einem Gehäuse davon verdreht werden kann, wenn die Drehhülse in einem vorgegebenen Lageverhältnis dazu ist, also z.B. vollständig eingeschoben ist. Hierzu kann z.B. eine umlaufende Aussparung, Rille oder Vertiefung auf der Drehhülse vorgesehen sein, welche nur im vollständig eingeschobenen Zustand der Drehhülse einem oder mehreren Rastnocken der Einstellhülse gegenüber liegt, die z.B. in auf der Innenseite eines Injektionsgehäuse oder eines anderen Elementes vorgesehene Rastöffnungen eingreifen und so ein Verdrehen der Einstellhülse relativ zur Injektionsvorrichtung bzw. zum Gehäuse der Injektionsvorrichtung verhindern. Nur wenn z.B. eine umlaufende Rille einer in der Einstellhülse geführten Drehhülse den Rastelementen der Drehhülse gegenüber liegt, können diese freigegeben werden und aus einem die Drehposition der Einstellhülse arretierenden Eingriff gelangen, so dass die Einstellhülse zur Einstellung einer Dosis gedreht werden kann. Wird die Drehhülse z.B. aus der Einstellhülse herausgedreht, also z.B. in axialer Richtung dazu versetzt, liegt die umlaufende Rille der Drehhülse nicht mehr den Rastelementen der Einstellhülse gegenüber, so dass diese in entsprechende Gegenrastelemente oder Eingriffe der Injektionsvorrichtung eingreifen und ein Verdrehen der Einstellhülse relativ zur Injektionsvorrichtung oder einem Gehäuse davon verhindern.

Gemäss einem weiteren Aspekt bezieht sich die Erfindung auf eine Dosiervorrichtung zur Einstellung einer aus einer Injektionsvorrichtung abzugebenden Dosis mit einer Einstellhülse zum Einstellen der Dosis z.B. durch Verdrehen der Einstellhülse relativ zur Injektionsvorrichtung, wobei die Einstellhülse nicht axial relativ zur Injektionsvorrichtung verändert wird, also nicht aus dieser herausfährt oder in diese eingeschoben wird. Erfindungsgemäss ist eine Drehhülse vorgesehen, welche so mit der Einstellhülse zusammengewirkt oder gekoppelt ist, dass diese nicht beim Einstellen einer Dosis proximal ausgefahren wird, sondern unabhängig von dem Einstellvorgang nach dem Einstellen der Dosis aus der Injektionsvorrichtung aus dem Gehäuse herausgedreht werden kann, um die Injektionsvorrichtung aufzuziehen. Hierdurch kann sichergestellt werden, dass der Einstellvorgang vom Aufziehvorgang der Injektionsvorrichtung entkoppelt werden kann oder unabhängig davon ist, so dass es beim Aufziehen der Injektionsvorrichtung z.B. nicht zu einer Fehl- oder Überdosierung kommen kann.

Weiterhin bezieht sich die Erfindung auf ein Verfahren zum Einstellen einer Dosis, welche aus einer Injektionsvorrichtung abgegeben werden soll, wobei die abzugebende Dosis mit einem Einstellelement, insbesondere einer Einstelldose, eingestellt werden kann und anschliessend die Injektionsvorrichtung mit einem Aufziehelement, insbesondere einer Drehhülse, aufgezogen werden kann, um in einem anschliessenden Schritt die eingestellte Dosis aus der Injektionsvorrichtung abzugeben, wobei der Einstellvorgang von dem Aufziehvorgang entkoppelt ist.

Die Erfindung bezieht sich gemäss einem weiteren Aspekt auf eine Dosiervorrichtung zur Einstellung einer aus einer Injektionsvorrichtung abzugebenden Dosis mit einer Drehhülse, welche aus der Injektionsvorrichtung zum Aufziehen der Injektionsvorrichtung herausgedreht werden kann und ein Innengewinde aufweist. Eine Gewindestange ist in der Drehhülse geführt und relativ zur Drehhülse bewegbar. Auf der Gewindestange ist ein Sperrelement, welches ein Aussengewinde aufweist, das in das Innengewinde der Drehhülse eingreifen kann, verschiebbar und verdrehgesichert gelagert und bis zu einem z.B. vorderen oder distalen Anschlagelement der Drehhülse vorschraubbar oder einschraubbar, um z.B. im vollständig vorgeschraubten Zustand eine Begrenzung der Aufziehbewegung der Drehülse zu bewirken. Ist die Gewindestange z.B. mittels einer ersten Verdreh- oder Rückdrehsicherung während eines Aufziehvorganges verdrehsicher in der Injektionsvorrichtung gelagert, wenn die Drehhülse aus der Injektionsvorrichtung herausgedreht wird, so kann bei entsprechender Ausbildung der Gewindegänge, also wenn z.B. das in das Innengewinde der Drehhülse eingreifende Aussengewinde des Sperrelements gleichläufig zum Aussengewinde der in der Injektionsvorrichtung eingeschraubten Drehhülse ist, das Sperrelement während eines Herausdrehvorganges der Drehhülse aus der Injektionsvorrichtung in proximaler Richtung relativ zur Drehhülse in distaler Richtung eingeschraubt werden. Wird die Drehhülse wieder in die Injektionsvorrichtung eingeschraubt, so bleibt die Relativposition zwischen Sperrelement und Drehhülse unverändert, wenn sich die Gewindestange zusammen mit der Drehhülse dreht, was z.B. durch eine zweite Verdreh- oder Rückdrehsicherung, die z.B. an der , Drehhülse vorgesehen oder mit dieser verbunden ist, realisiert werden kann. Durch das distale Hineinschrauben des Sperrelements in die Drehhülse während eines Aufziehvorganges der Drehhülse kann festgelegt werden, welche Gesamtmenge einer Substanz mittels der Dosiervorrichtung aus der Injektionsvorrichtung abgegeben werden kann, bevor das Sperrelement z.B. vollständig in die Drehhülse eingeschraubt ist oder an eine Anschlagsposition gelangt, was ein weiteres Herausziehen der Drehhülse verhindert, wenn die Drehhülse verdrehgesichert auf der Gewindestange gelagert ist und diese während des Aufziehvorganges drehsicher z.B. durch eine erste Verdrehsicherung in der Injektionsvorrichtung gehalten wird. Somit kann das Sperrelement bei der letzten Dosiseinstellung die Drehhülse und damit Einstellungen blockieren, die über der noch zur Verfügung stehenden Menge der abzugebenden Substanz liegen.

Wird die Anfangs- oder Startposition des Sperrelementes auf der Gewindestange oder in der Drehhülse so festgelegt, dass die zum distalen Eindrehen des Sperrelements bis zu einem Anschlag benötigten Aufziehvorgänge nicht über die in der Injektionsvorrichtung enthaltene zugeordnete Substanzmenge hinausgehen öder dieser entsprechen, kann sichergestellt werden, dass keine Dosis aufgezogen werden kann, welche nicht mehr aus der Injektionsvorrichtung abgegeben werden kann. Somit können Fehldosierungen bei der Abgabe der letzten Dosis aus der Injektionsvorrichtung vermieden werden und ein Benutzer kann z.B. anhand der durch das Sperrelement blockierten maximalen letzten Aufziehposition ermitteln, welche Menge der Substanz er noch aus der Injektionsvorrichtung abgeben kann, um z.B. die Injektionsvorrichtung nicht mehr zu verwenden oder z.B. die restliche Menge der abzugebenden Substanz aus der Injektionsvorrichtung abzugeben und die für eine Behandlung fehlende Restmenge aus einer anderen Injektionsvorrichtung oder anderen Ampulle abzugeben.

Vorzugsweise sind das Gewinde der Gewindestange und das Aussengewinde des Sperrelementes gleichläufig.

Eine Verdreh- oder Rückdrehsicherung wird nachfolgend erläutert und ermöglicht ein Verdrehen zum Beispiel der Gewindestange relativ zu der Injektionsvorrichtung oder zur Drehhülse in eine Richtung und blockiert eine Drehung in Gegenrichtung. Die Drehsicherungen an der Injektionsvorrichtung und der Drehhülse können gleich aufgebaut sein und den gleichen Wirkmechanismus im Sinne einer Ratsche haben.

Die Erfindung bezieht sich weiterhin auf ein Verfahren zum Begrenzen einer maximal möglichen Dosis oder Einstellmenge einer Injektionsvorrichtung, wobei ein Sperrelement während eines Aufziehvorganges der Injektionsvorrichtung vor dem Abgabevorgang der aus der Injektionsvorrichtung abzugebenden Dosis in Richtung auf einen Anschlag oder eine Sperr- oder Blockierposition bewegt wird und in Abhängigkeit von der Entfernung des Sperrelementes von der Sperr- oder Blockierposition die maximale Menge der mit einer Dosiervorrichtung aufzuziehenden oder aus der Injektionsvorrichtung abzugebenden Substanz eingestellt wird.

Gemäss einem weiteren Aspekt bezieht sich die Erfindung auf eine Gewindestange, die einen Druck auf einen Stopfen in einem Reservoir einer Injektionsvorrichtung ausüben kann, welches eine abzugebende Substanz enthält und auf eine Dosiervorrichtung zur Einstellung einer aus einer Injektionsvorrichtung abzugebenden Dosis oder Substanz mit einer solchen Gewindestange.

Die Injektionsvorrichtung weist eine Drehhülse auf, mit welcher eine aus der Injektionsvorrichtung abzugebende Dosis durch Drehung der Drehhülse, also z.B. durch ein Herausschrauben oder Einschrauben der Drehhülse in die Injektionsvorrichtung, aufgezogen oder eingestellt werden kann. Die Gewindestange trägt an ihrer Aussenseite ein Gewinde, welches als umlaufendes Gewinde ausgebildet sein kann oder aus mehreren z.B. axial und/oder in Umfangsrichtung versetzten Gewindeteilstücken bestehen kann, und ist bevorzugt in der Injektionsvorrichtung und/oder der Einstellhülse geführt und in diesen axial bewegbar, z.B. durch ein auf der Innenseite der Injektionsvorrichtung oder Einstellhülse vorgesehenes Drehbegrenzungselement. Das Gewinde der Gewindestange kann von dieser vorstehen z.B. als Steg oder vertieft z.B. als Nut ausgebildet sein.

An der Injektionsvorrichtung und/oder der Einstell- und/oder der Drehhülse ist ein z.B. mit dem Gehäuse der Injektionsvorrichtung fest verbundenes Drehbegrenzungselement vorgesehen, welches als Drehraster, Verdrehsicherung oder Rückdrehsicherung ausgebildet sein kann. Ein Drehbegrenzungselement im Sinne der Erfindung ist ein Element, welches im Zusammenwirken mit einem durch oder in dem Drehbegrenzungselement geführten Element, wie z.B. der Gewindestange, eine Drehung des geführten Elements, wie z.B. der Gewindestange, nur in eine Richtung ermöglicht und in die entgegengesetzte Richtung sperrt oder verhindert. Hierzu können auf der Gewindestange entsprechende Gegenelemente vorgesehen sein. Beispielsweise kann mit einer Kombination von Rastnasen oder -nocken auf dem Drehbegrenzungselement oder der Gewindestange, welche in entsprechende Einraststellen, wie z.B. eine Rasterung oder Zahnung, der Gewindestange oder des Drehbegrenzungselementes eingreifen, eine Drehbegrenzung realisiert werden, welche z.B. nach dem Prinzip einer bekannten Ratsche funktioniert. Eine Rastnase kann beispielsweise auf einem z.B. in radiale Richtung vorgespannten elastischen Element, wie z.B. einem Rastarm, der in Umfangsrichtung des geführten Elementes auf diesem oder dem Drehbegrenzungselement angeordnet ist, angeordnet sein oder direkt auf dem jeweiligen Element ohne elastisches Element angeordnet sein und z.B. eine Abschrägung auf einer Seite aufweisen, um ein Herausgleiten aus einer Einraststelle zu ermöglichen und eine der abgeschrägten Seite gegenüberliegenden nicht abgeschrägten Seite haben, welche eine Drehung in der zum Herausgleiten des Rastelements erforderlichen entgegengesetzten Richtung verhindert.

Das auf der Gewindestange vorgesehene Gewinde kann z.B. eine Zahnung auf dem Gewindegang oder Gewindesegment aufweisen, in welche ein oder mehrere Rastelemente eines oder mehrerer Drehbegrenzungselemente eingreifen können, um so eine Drehung der Gewindestange relativ zum Drehbegrenzungselement nur in eine Richtung zu ermöglichen. Eine Gewindestange kann also z.B. in eine mit einem Drehbegrenzungselement verbundene Einstellhülse nur eingeschraubt oder nur herausgedreht werden, wobei die jeweilige Gegendrehbewegung durch das Drehbegrenzungselement blockiert wird.

Die Injektionsvorrichtung weist bevorzugt ebenso wie die Drehhülse ein Drehbegrenzungselement auf, welches eine Drehung einer Gewindestange in die gleiche Richtung ermöglicht und in die entgegengesetzte Richtung blockiert. Da die Drehhülse drehbar und bevorzugt durch ein Gewinde geführt in der Injektionsvorrichtung gelagert ist und aus dieser zum Aufziehen der Injektionsvorrichtung herausgezogen oder herausgeschraubt und zum Abgeben einer Dosis wieder eingeschoben oder eingeschraubt werden kann, ermöglicht es eine an der Drehhülse vorgesehene Rückdrehsicherung, dass während des Herausdrehens der Drehhülse aus der Injektionsvorrichtung die Gewindestange von der Drehsicherung der Injektionsvorrichtung gehalten wird, während die Drehsicherung der Drehhülse eine Herausdrehbewegung aus der Injektionsvorrichtung ermöglicht. Wird die Drehhülse wieder in die Injektionsvorrichtung eingeschraubt, so greift das Drehbegrenzungselement der Drehhülse in die Gewindestange ein und bewirkt, dass die Drehhülse die Einschraubbewegung in die Injektionsvorrichtung auf die Gewindestange überträgt und diese mitnimmt, wobei ein Mitdrehen der Gewindestange durch das Drehbegrenzungselement der Injektionsvorrichtung ermöglicht wird, weiches eine Drehung der Gewindestange während des Herausschraubens der Drehhülse aus der Injektionsvorrichtung blockiert hat.

Durch ein Mitdrehen der Gewindestange während des Eindrehens der Drehhülse kann die zum Beispiel in einem Gewinde der Injektionsvorrichtung geführte Gewindestange in der Injektionsvorrichtung distal verschoben werden, um z.B. einen Stopfen eine definierte Strecke in ein Behältnis, wie z.B. eine Ampulle, einzuschieben und so ein in dem Behältnis enthaltenes Fluid oder eine Substanz aus diesem zu verdrängen und so durch eine Abgabeöffnung des Behältnisses z.B. an einen Patienten abzugeben.

An der Drehhülse kann ein Drehknopf angebracht sein, welcher relativ zur Drehhülse gedreht werden kann, so dass ein Benutzer z.B. die Drehhülse mittels eines Zuges an dem Drehknopf aus der Injektionsvorrichtung herausziehen oder herausdrehen kann, ohne dass sich der Drehknopf relativ zur Injektionsvorrichtung dreht, wobei die Drehhülse drehbar in dem Drehknopf gehalten wird. Ebenso ist ein Einschieben oder Eindrehen bzw. Einschrauben der Drehhülse in die Injektionsvorrichtung durch einen Druck auf den Drehknopf möglich, ohne dass sich der Drehknopf relativ zur Injektionsvorrichtung bewegt.

Die Erfindung wird nachfolgend anhand bevorzugter Ausführungsbeispiele beschrieben. Es zeigen:
- Fig. 1A: eine Seitenansicht einer erfindungsgemässen Injektionsvorrichtung;
- Fig. 1B: eine Schnittansicht entlang der Linie B-B in Fig. 1A;
- Fig. 1C: eine Draufsicht auf die in Fig. 1A gezeigte Injektionsvorrichtung;
- Fig. 1D: eine Schnittansicht entlang der Linie D-D in Fig. 1C;
- Fig. 2A - 2H: die Stellungen der Dosiervorrichtung beim Einstellen einer Dosis, Aufziehen der Injektionsvorrichtung und der Abgabe einer Dosis;
- Fig. 4A und: 3Beine erste Ausführungsform einer erfindungsgemässen Drehsicherung; und

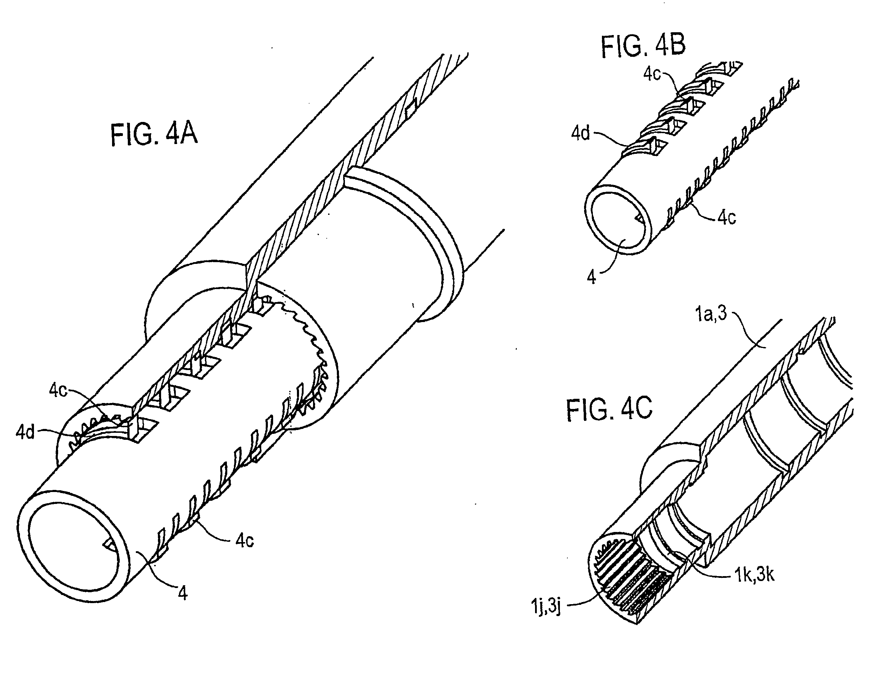

- Fig. 4A - 4C: eine weitere Ausführungsform einer erfindungsgemässen Drehsicherung.

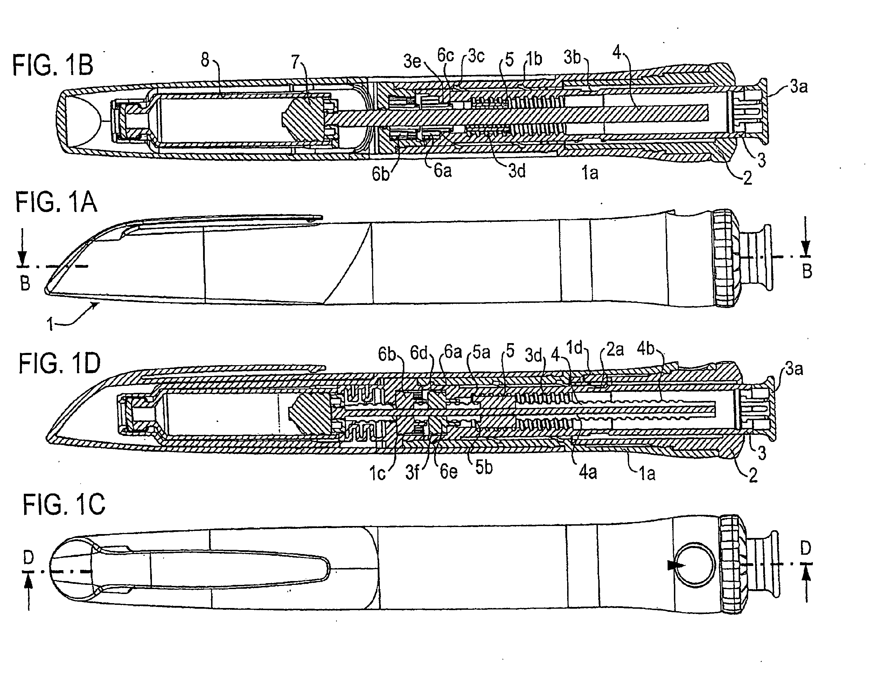

Fig. 1A zeigt eine Seitenansicht einer Injektionsvorrichtung (1), deren Schnitt entlang der Linie B-B in Fig. 1B dargestellt ist.

Die Injektionsvorrichtung (1) weist ein Gehäuse oder Gehäuseelement (1a) auf, in welchem eine Einstellhülse (2) zum einmaligen Vordosieren oder Einstellen der Injektionsvorrichtung z.B. durch einen Arzt oder auch zum mehrmaligen Einstellen verschiedenen Dosismengen drehbar angeordnet ist. Innerhalb der Einstellhülse (2) ist eine Drehhülse (3) drehbar gelagert, welche ein Aussengewinde (3c) hat, das in ein Innengewinde (1b) der Injektionsvorrichtung (1) oder eines Gehäuseteiles (1a) der Injektionsvorrichtung eingreift. Die Drehhülse (3) weist ein Innengewinde (3d) auf, in welches ein Aussengewinde (5a) eines Sperrelementes 5 eingreift, so dass die Drehhülse (3) im Gewindeeingriff mit der Injektionsvorrichtung (1) bzw. einem Gehäuse (1a) davon und im Gewindeeingriff mit dem Sperrelement (5) ist. Das Sperrelement (5) ist verdrehgesichert aber axial verschiebbar auf einer Gewindestange (4) gelagert und weist zwei gegenüberliegende Führungselemente (5b) auf, die in eine in axialer Richtung längs der Gewindestange (4) verlaufende Führungsrille (4a) eingreifen, so dass eine Drehung der Gewindestange (4) immer auf das Sperrelement (5) übertragen wird, welches sich mit der Gewindestange (4) mitdreht bzw. welches auch eine Drehung der Gewindestange 4 blockieren kann, wenn es z.B. vollständig in die Drehhülse 3 eingeschraubt ist.

Am distalen Ende der Drehhülse (3) ist ein Drehbegrenzungselement (6a) vorgesehen, welches so mit der Drehhülse (3) verbunden ist, dass sich das Drehbegrenzungselement (6a) in axialer Richtung nicht von der Drehhülse (3) lösen kann, wobei das Drehbegrenzungselement (6a) durch Schnapper oder Halteelemente (6c) so von einem Mitnehmer (3e) der Drehhülse (3) gehalten wird, dass das Drehbegrenzungselement (6a) zwar drehbar relativ zur Drehhülse (3) gelagert ist, jedoch in axialer Richtung nicht relativ zur Drehhülse (3) verschoben werden kann.

Das Drehbegrenzungselement (6a) weist radial nach aussen durch Federarme vorgespannte Rastelemente (6d) auf, die in eine umlaufend an der Innenseite des distalen Endes der Drehhülse (3) vorgesehene Zahnung oder Rasterung (3f) eingreifen, wobei die Zahnung (3f) und die Rastelemente (6d) so ausgebildet sind, dass eine Drehung des Drehbegrenzungselementes (6a) in der Drehhülse (3) nur in einer Richtung möglich ist, in welcher die Rastelemente (6d) aus dem Eingriff mit der Zahnung (3f) herausgleiten können, während eine Drehung in die Gegenbewegung durch eine entsprechende Ausbildung der Rastelemente (6d) verhindert wird, welche in die Zahnung (3f) eingreifen und eine Gegenrichtung blockieren, wobei von dem einseitigen Drehbegrenzungseffekt einer an sich bekannten Ratsche Gebrauch gemacht wird.

Das Drehbegrenzungselement (6a) weist ebenso wie das Sperrelement (5), einander gegenüberliegende Führungselement (6e) auf, welche in die Axialführung oder Rille (4a) der Gewindestange (4) eingreifen und so relativ zur Gewindestange (4) verdrehgesichert sind, wobei jedoch die Geweindestange (4) axial relativ zum Drehbegrenzungselement (6a) verschoben werden kann.

Ein weiteres Drehbegrenzungselement (6b), welches identisch wie das Drehbegrenzungselement (6a) aufgebaut ist, ist drehbar in der Injektionsvorrichtung (1) gelagert und greift mit Rastelementen (6d) in eine auf der Innenseite eines Injektionsvorrichtungsteiles umlaufende Zahnung (1 c) ein, so dass das Drehbegrenzungselement (6b) in axialer Richtung nicht in der Injektionsvorrichtung (1) verschoben werden kann und nur eine Drehung in einer Richtung der Injektionsvorrichtung (1) ermöglicht und in der entgegengesetzten Richtung sperrt.

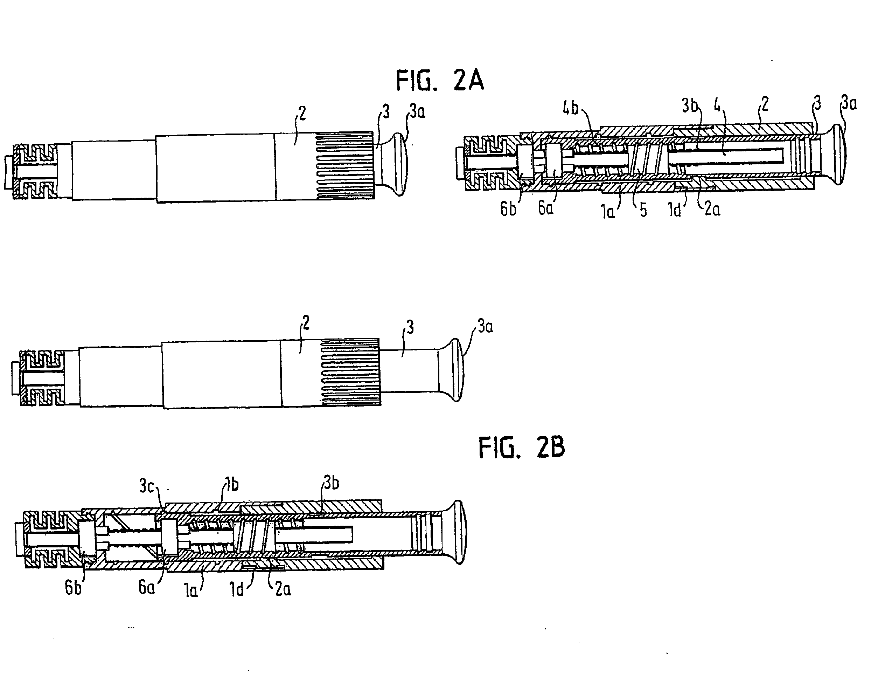

Die prinzipielle Funktionsweise einer Ausführungsform einer erfindungsgemässen Injektionsvorrichtung wird unter Bezugnahme auf die in den Fig. 2A - 2H gezeigten Zustände einer Ausführungsform einer erfindungsgemässen Dosiervorrichtung erläutert. Fig. 2A zeigt in Draufsicht und Querschnittsansicht eine Dosiervorrichtung mit einer Gewindestange (4), welche in der gezeigten Ausführungsform abgeflacht und im Querschnitt etwa rechteckig ist und Gewindesegmente 4b an zwei gegenüberliegenden Seitenflächen trägt, zwischen welchen Teilstücke der Gewindestange (4) liegen, auf welchen keine Gewindeelemente vorgesehen sind. Eine verdrehgesicherte Lagerung der Gewindestange (4) in dem Sperrelement (5) oder den Drehbegrenzungselementen (6a, 6d) wird dadurch erreicht, dass in diesen Elementen etwa der Querschnittsform der Gewindestange (4) entsprechende Durchgangsöffnungen vorgesehen sind.

Durch Drehen an der Drehhülse (3) wird eine gewünschte aufzuziehende und abzugebende Dosis eingestellt. Der Einstellsperrnocken (2a) der Einstellhülse (2) ist nach innen freigestellt und kann in die Freigaberille (3b) geschoben werden, um die Kopplung der Einstellhülse (2) mit dem Gehäuse (1a) durch einen Eingriff des Einstellsperrnockens (2a) in auf der Innenseite des Gehäuses (1a) liegende Rasterungen aufzuheben.

Nach dem Einstellen der Dosis mittels der frei drehbaren Einstellhülse (2) wird die Injektionsvorrichtung aufgezogen, wie in Fig. 2B in Draufsicht und in Querschnittsansicht gezeigt. Durch Ziehen am frei drehbaren Drehknopf (3a) wird die Injektionsvorrichtung mit der vorher mittels der Einstellhülse (2) eingestellten Dosis von z.B. 2.6 Einheiten aufgezogen oder geladen. Die Drehhülse (3) wird zusammen mit dem frei drehbaren Drehknopf (3a) in proximale Richtung (in Fig. 2B nach rechts) gezogen und dreht sich aufgrund des Gewindeeingriffs des Aussengewindes (3c) der Drehhülse (3) im Innengewinde (1c) des Injektionsvorrichtungsgehäuses (1a).

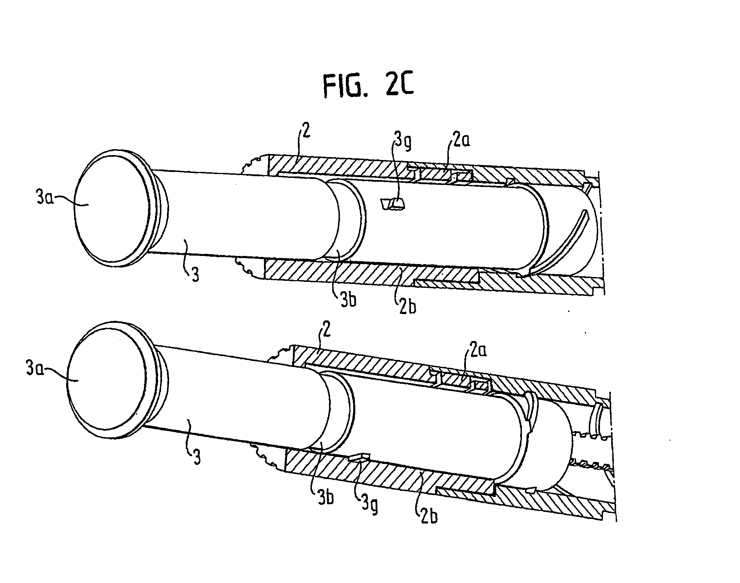

Eine auf der Innenseite der Einstellhülse (2) vorgesehene Anschlagleiste (2b), deren radiale Position durch den Einstell- bzw. Drehvorgang der Einstellhülse (2) festgelegt wurde, begrenzt eine maximal mögliche Dreh- oder Aufziehbewegung der Drehhülse (3), welche auf ihrer Aussenseite ein Anschlagelement (3g) z.B. in Form eines vorstehenden Nockens trägt. Wie in Fig. 2C gezeigt, kann die Drehhülse (3) soweit aus der Injektionsvorrichtung (1) herausgedreht werden, bis das Anschlagelement (3g) an der Anschlagsleiste (2b) der Einsteilhülse anliegt und somit ein weiteres Drehen oder Herausziehen der Einstellhülse (2) verhindert. Wie aus den Fig. 2B und 2C ersichtlich ist, wird während eines Aufziehvorganges beim Herausziehen der Drehhülse (3) die auf der Drehhülse (3) vorgesehene Freigaberille (3b) von dem Einstellsperrnocken (2a) der Einstellhülse (2) wegbewegt, so dass der Einstellsperrnocken (2a) nicht mehr radial nach innen ausweichen kann und somit durch einen Eingriff in die auf der Innenseite des Gehäuses (1a) vorgesehene Rasterung (1d) eingreift und somit eine radiale oder Verdrehbewegung der Einsteilhülse (2) relativ zur Injektionsvorrichtung (1) bzw. dem Gehäuseelement (1a) verhindert. Eine Veränderung der mittels der Einstellhülse (2) einstellbaren Dosis kann somit während eines Aufzieh- und Abgabevorganges bei zumindest zum Teil herausgezogener Drehhülse (3) nicht mehr vorgenommen werden.

Im Inneren der Einstellhülse (2) dreht sich während eines Aufziehvorganges das Sperrelement (5) relativ zur Einstellhülse (2) in dieses hinein, d.h. das Sperrelement wird relativ zur Einstellhülse (2) in distale Richtung der Einstellhülse (2) bewegt, da das Sperrelement verdrehgesichert auf der Gewindestange (4) gelagert ist und die Drehhülse (3) beim Herausdrehen relativ zum Sperrelement (5) gedreht wird, was durch den Gewindeeingriff des Sperrelementes (5) in das Innengewinde (3d) der Drehhülse (3) beim Herausdrehen für eine Relativbewegung des Sperrelementes (5) in axialer Richtung der Drehhülse (3) sorgt.

Während des Aufziehvorganges der Injektionsvorrichtung, d.h. während des Herausdrehens der Drehhülse (3), verhindert das vordere mit der Injektionsvorrichtung verbundene Drehbegrenzungselement (6b), dass die Gewindestange (4) beim Aufziehen der Injektionsvorrichtung mitgedreht wird, während das mit der Drehhülse (3) verbundene Drehbegrenzungselement (6a) eine Drehbewegung der Drehhülse (3) relativ zur durch das Drehbegrenzungselement (6b) gehaltenen Gewindestange (4) ermöglicht.

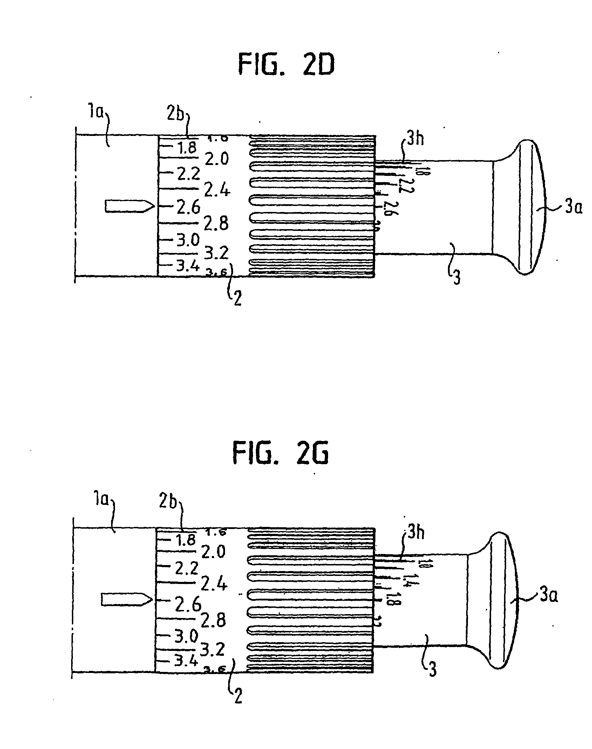

Wie in Fig. 2D gezeigt, ist auf der Aussenseite der Einstellhülse (2) in Umfangsrichtung eine Markierung vorgesehen, wobei in der in Fig. 2D gezeigten Einstellung eine Dosis von 2.6 eingestellt wurde, indem die Einstellhülse (2) so gedreht wird, dass eine der Dosis 2.6 durch einen Strich markierte zugeordnete Drehposition an einem durch einen Pfeil dargestellten Markierungselement auf dem Gehäuse (1a) liegt. Im vollständig herausgezogenen Zustand der Drehhülse (3), welche durch den Anschlag des Anschlagelementes (3g) an der Anschlagleiste (2b) bestimmt wird, ist die Drehhülse (3) so weit aus der Injektionsvorrichtung herausgedreht, dass eine auf der Umfangsseite der Drehhülse (3) vorgesehene Markierung der tatsächlich aufgezogenen Dosis 2.6 vollständig zu erkennen ist, wobei die Markierung 2.6 auf der Drehhülse (3) in axialer Verlängerung zur Markierung 2.6 auf der Einstellhülse (2) liegt, um einem Benutzer so anzuzeigen, dass die eingestellte Dosis auch tatsächlich aufgezogen wurde. Ein Benutzer kann einen Vergleich der eingestellten mit der aufgezogenen Dosis durch einen Blick auf die Injektionsvorrichtung vornehmen, ohne eine z.B. auf einer Seite der Injektionsvorrichtung angezeigte eingestellte Dosis mit einer auf einer anderen Seite der Injektionsvorrichtung angezeigte "tatsächlich aufgezogene" Dosis vornehmen zu müssen.

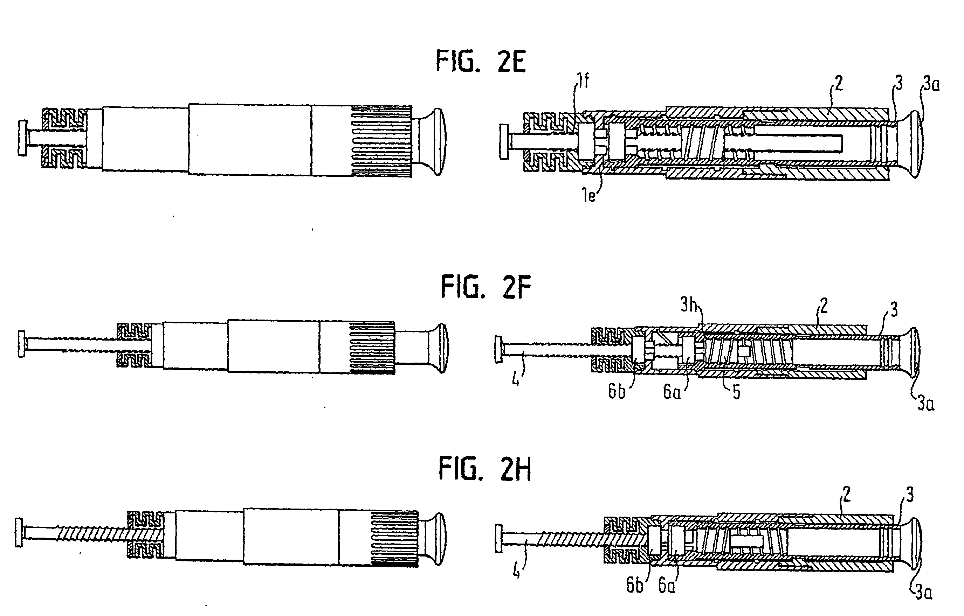

Zum Ausschütten einer voreingestellten Dosis kann der Drehknopf (3a) gedrückt werden, wodurch die drehbar in dem Drehknopf (3a) gelagerte Drehhülse (3) in die Injektionsvorrichtung eingedreht wird, wie in Fig. 2E gezeigt, bis ein vorderes oder distales Ende der Einstellhülse (2) an einem Innenanschlag (1e) der Injektionsvorrichtung (1) anliegt. Beim Eindrehen der Einstellhülse (2) gibt das mit der Injektionsvorrichtung (1) verbundene vordere Drehbegrenzungselement (6b) die Drehbewegung der Gewindestange (4) frei, welche von dem mit der Drehhülse (3) verbundenen Drehbegrenzungselement (6a) gehalten und zusammen mit der Drehhülse (3) relativ zur Injektionsvorrichtung (1) gedreht wird und sich so durch einen Gewindeeingriff in ein Innengewinde (1f) der Injektionsvorrichtung (1) relativ zur Injektionsvorrichtung (1) in distale Richtung schraubt, um einen z.B. an der Vorderseite der Gewindestange (4) anliegenden Stopfen (7), wie in Fig. 1 B gezeigt, in eine Ampulle (8) einzuschieben und eine in der Ampulle (8) enthaltene Substanz zu verdrängen und so die voreingestellte Dosis abzugeben.

Nach erfolgtem Abgabevorgang kann die mittels der Drehhülse (3) eingestellte Dosis einfach wieder durch Herausziehen der Drehhülse (3) aufgezogen und abgegeben werden, wie oben beschrieben, ohne dass eine erneute Dosiseinstellung vorgenommen werden muss, da die Einstell- oder Drehposition der Einstellhülse (2) während des Aufzieh- und Abgabevorganges nicht verändert wurde.

Wird das Sperrelement (5) so in die Drehhülse (3) eingesetzt, dass die insgesamt mögliche von dem Sperrelement (5) bis zu einem vorderen Anschlag innerhalb der Drehhülse (3) zurückzulegende Strecke einer gewünschten maximal abzugebenden Substanzmenge entspricht, so kann, falls eine mittels der Einstellhülse (2) eingestellte Dosis grösser als die vorgegebene maximal zulässige Gesamtabgabemenge ist, eine Begrenzung des Aufziehvorganges der Injektionsvorrichtung durch ein Blockieren des Herausdrehens der Drehhülse (3) realisiert werden.

Wird z.B. eine letzte zulässige Dosis aufgezogen, wie in Fig. 2F gezeigt, so wird wiederum die Drehhülse (3) mittels des Drehknopfes (3a) aus der Injektionsvorrichtung (1) herausgezogen bzw. herausgedreht. Gleichzeitig dreht sich im Inneren der Drehhülse (3) das Sperrelement (5) so weit in die Drehhülse (3) hinein, bis das Sperrelement (5) an einem vorderen Anschlag (3h) der Drehhülse (3) liegt und so ein weiteres Herausdrehen der Drehhülse (3) blockiert, die durch den Gewindeeingriff mit dem Aussengewinde (5a) des Sperrelementes (5) gehalten wird, welches sich relativ zur Injektionsvorrichtung nicht drehen kann, da es verdrehsicher auf der Gewindestange (4) gelagert ist, die während des Herauszieh- oder Aufziehvorganges drehsicher von dem vorderen mit der Injektionsvorrichtung(1) verbundenen Drehbegrenzungselement (6b) gehalten wird.

Wie in Fig. 2G gezeigt, kann ein Benutzer leicht erkennen, dass in axialer Verlängerung der auf der Einstellhülse (2) vorgesehenen Markierung 2.6 im blockierten herausgedrehten Endzustand der Drehhülse (3) die maximal noch mögliche aufgezogene Dosis 1.8 angezeigt wird, so dass ein Benutzer leicht ablesen kann, dass die gewünschte eingestellte Dosis nicht mehr abgegeben werden kann.

Wie in Fig. 2H gezeigt, kann durch Drücken auf den Drehknopf (3a) die letzte Dosis ausgeschüttet werden. Durch das vollständig in die Drehhülse (3) eingeschraubte Sperrelement (5), welches an dem vorderen Anschlag (3h) der Drehhülse (3) ansteht, wird verhindert, dass die Injektionsvorrichtung nochmals aufgezogen werden kann.

Durch den Unterschied der Gewindesteigungen des Aussengewindes (4b) der Gewindestange (4) und des Aussengewindes (3c) der Drehhülse (3) kann, wenn z.B. die Gewindesteigung des Aussengewindes (3c) der Drehhülse (3) grösser als die des Aussengewindes (4b) der Gewindestange (4) ist, eine Untersetzung des Abgabevorganges realisiert werden. Die Drehhülse (3) wird während des Aufziehens weiter aus der Injektionsvorrichtung (1) herausgezogen, als die Gewindestange (4) axial in distale Richtung beim Einschieben der Drehhülse (3) verschoben wird, da während des Einschiebe- oder Abgabevorganges die Drehhülse (3) mit der Gewindestange (4) drehgekoppelt ist und beide Element somit die gleiche Drehbewegung ausführen. Da das Aussengewinde (3c) der Drehhülse (3) eine grössere Steigung aufweist als das Aussengewinde (4b) der Gewindestange (4), kann mit einer in axialer Richtung grösseren Aufzieh- und Einschubbewegung der Drehhülse (3) eine in axialer Richtung kleinere Vorschubbewegung der. Gewindestange (4) bewirkt werden, was zu einer feineren Dosierung der durch eine Vorschubbewegung der Gewindestange (4) aus einer Ampulle (8) abgegebenen Substanz führt. Ebenso kann das Verhältnis der Steigung des Aussengewindes der Drehhülse (3) zu der des Aussengewindes dem Gewindestange 4 so festgelegt werden, dass eine Übersetzung oder auch keine Über- oder Untersetzung stattfindet.

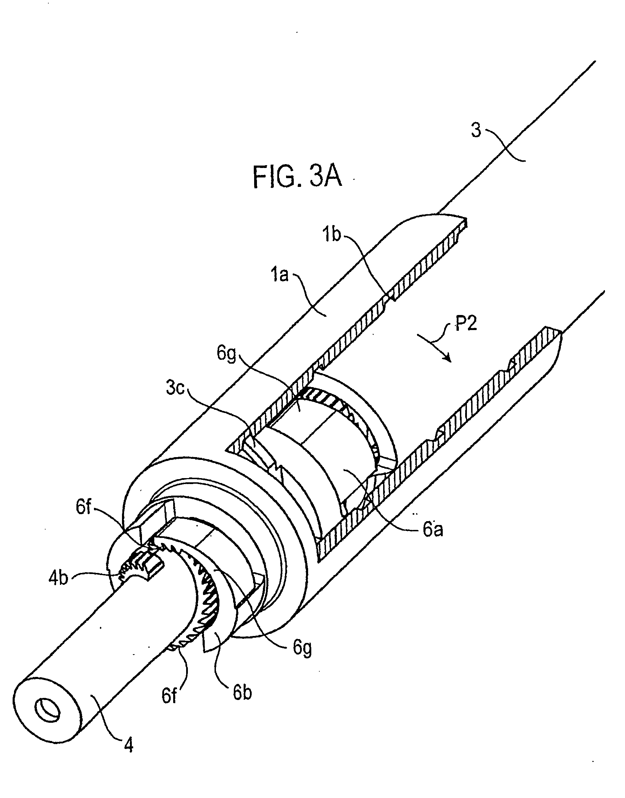

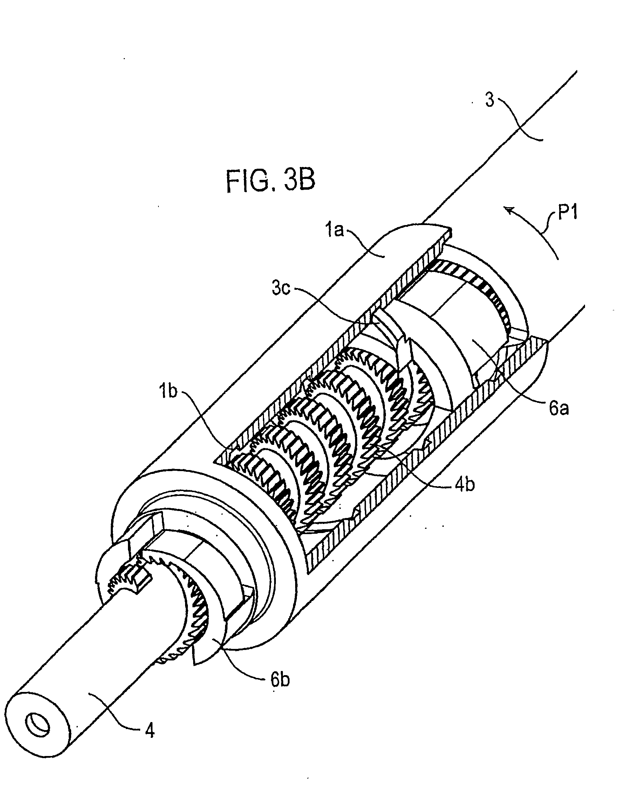

Die Fig. 3A und 3B zeigen eine weitere Ausführungsform der Drehbegrenzungselemente 6a und 6b. In der gezeigten Ausführungsform weist die Gewindestange (4) ein umlaufendes Gewinde (4b) auf, welches in Umfangsrichtung eine Zahnung trägt, bei welcher die einzelnen Zähne so ausgebildet sind, dass an einer Zahnflanke ein Rasten oder Halten mit einer entsprechenden Gegenzahnung oder einem Rastelement möglich ist, während die andere Zahnflanke so ausgebildet ist, dass an einem Rast- oder Gegenelement eine Gleit- oder Verschiebebewegung möglich ist. Beispielsweise kann die ein Eingreifen oder Halten bewirkende Zahnflanke im Wesentlichen in radialer Richtung nach aussen verlaufen, während die eine Dreh- oder Gleitbewegung ermöglichende Zahnflanke relativ zur dieser Zahnflanke schräg oder geneigt ist.

Mit der Injektionsvorrichtung (1) oder einem Gehäuseteil (1a) davon fest verbunden oder darin integriert ist ein Drehbegrenzungselement (6b), weiches in der gezeigten Ausführungsform zwei durch jeweils drei Zahnflanken gebildete Rastelemente (6f) aufweist, welche einander gegenüber liegen und an elastischen oder Rastarmen (6g) angebracht sind. Das Gewinde (4b) wird durch ein Innengewinde innerhalb der Injektionsvorrichtung (1) bzw. des Gehäuseteiles (1 a) geführt, so dass die Gewindestange (4) durch einen Gewindeeingriff drehbar in der Injektionsvorrichtung (1) bzw. dem Gehäuseteil (1a) gelagert ist und in dieses ein- bzw. ausgeschraubt werden kann. Durch den Eingriff der Rastelemente (6f) in die auf dem Gewinde (4b) der Gewindestange (4) vorgesehene Zahnung ist eine Drehung der Gewindestange (4) relativ zum Gehäuseteil (1a) nur in einer Richtung möglich, wohingegen eine Bewegung in die entgegengesetzte Richtung blockiert wird. In der in Fig. 3A gezeigten Ausführungsform kann die Gewindestange (4) im Uhrzeigersinn gedreht werden, wodurch die Gewindestange (4) in distaler Richtung aus dem Gehäuseteil (1a) nur herausgeschraubt aber nicht in entgegengesetzter Richtung eingeschraubt werden kann.

Ebenso wie das Drehbegrenzungselement (6b) ist das Drehbegrenzungselement (6a) aufgebaut, welches fest mit der Drehhülse (3) verbunden oder in diese integriert ist und ebenfalls Rastelemente (6f) aufweist, die an elastischen um die Gewindestange (4) umlaufenden Armen (6g) angebracht sind und in die Zahnung des Gewindes (4b) der Gewindestange (4) eingreifen können, um, wie oben beschrieben, eine Drehbewegung zwischen Gewindestange (4) und Drehhülse (3) in einer Richtung zu ermöglichen und in der entgegengesetzten Richtung zu blockieren.

Fig. 3A zeigt die vollständig in die Injektionsvorrichtung eingedrehte Drehhülse (3), welche über ein Aussengewinde (3c) in einem Innengewinde (1 b) des Injektionsgerätegehäuses (1a) geführt ist.

Wird die Injektionsvorrichtung (1) durch ein Herausdrehen der Drehhülse (3) aufgezogen, so wird die Gewindestange (4) von dem Drehbegrenzungselement (6b) verdrehsichert relativ zum Injektionsgerätegehäuses (1a) gehalten, während das Drehbegrenzungselement (6a) bei der in der durch den Pfeil P1 angegebenen Drehrichtung, welche ein Herausdrehen der Drehhülse (3) aus dem Gehäuse (1a) bewirkt, nicht in Blockiereingriff mit der Zahnung auf dem Gewindegang (4b) der Gewindestange (4) ist.

Beim Wiedereindrehen der Drehhülse (3) in der in Fig. 3A durch den Pfeil P2 gezeigten Richtung sind die Eingriffselemente des Drehbegrenzungselementes (6a) der Drehhülse (3) im Eingriff mit der Zahnung des Gewindes (4a) der Gewindestange 4, wodurch während des Eindrehens der Drehhülse (3) die Gewindestange (4) drehsicher mit der Drehhülse (3) gekoppelt ist und von dieser mitgenommen und mitgedreht wird. Das Drehbegrenzungselement (6b) der Injektionsvorrichtung (1) ermöglicht eine Drehung der Gewindestange (4) relativ zur Injektionsvorrichtung (1) bzw. dem Gehäuse (1a), so dass die Gewindestange (4) durch die Drehhülse (3) gedreht und in dem Innengewinde der Injektionsvorrichtung (1) bzw. dem Gehäuse (1 a) geführt wird und sich so aus dem Gehäuse (1 a) in distale Richtung herausdreht, um eine eingestellte und aufgezogene Dosis abzugeben. Anschliessend kann die Injektionsvorrichtung durch ein Herausdrehen der Drehhülse (3) in der durch den Pfeil P1 in Fig. 3B angegebenen Richtung wieder aufgezogen werden.

Die in den Fig. 3A und 3B gezeigte Ausführungsform der Drehbegrenzungselemente 6a und 6b kann anstatt der unter Bezugnahme auf die Fig. 1 und 2 beschriebenen Drehbegrenzungselemente 6a und 6b in einer Dosiervorrichtung oder Injektionsvorrichtung verwendet werden, wobei alle weiteren Elemente der Injektionsvorrichtung unverändert bleiben können. Z.B. kann das Sperrelement (5) verdrehsicher auf der Gewindestange (4) geführt werden, indem ein oder mehrere Eingriffselemente in axialer Richtung auf der Innenseite des Sperrelementes (5) vorgesehen sind, welche in der Zahnung des Aussengewindes (4b) der Gewindestange (4) geführt werden können, um das Sperrelement (5) axial verschieblich aber drehgesichert auf der Gewindestange (4) zu lagern.

Die Fig. 4A - 4C zeigen eine weitere Ausführungsform eines Drehbegrenzungselementes 6a oder 6b, welches an einem Gehäuse (1a) der Injektionsvorrichtung oder an der Drehhülse (3) oder in diese Elemente integriert sein kann. Dabei ist der Wirkmechanismus im Vergleich unter Bezugnahme auf die in Fig. 3A und 3B beschriebene Ausführungsform umgekehrt. Auf der Gewindestange (4) sind Gewindeabschnittselemente (4c) vorgesehen, welche durch elastische und z.B. radial nach aussen vorgespannte Tragarme (4d) an der Gewindestange (4) befestigt sind und in eine Innenzahnung (1j oder 3j) des Injektionsvorrichtungsgehäuses (1 a) oder der Drehhülse (3) eingreifen können. Auf der Innenseite des Gehäuses (1a) bzw. der Drehhülse (3) ist axial zu der Rasterung oder Zahnung 1j, 3j versetzt ein Gewinde oder Gewindeabschnitt (1k, 3k) vorgesehen, in welchem das durch die Rastelemente (4c) und Tragarme 4d gebildete Aussengewinde der Gewindestange (4) geführt werden kann.

Die Gewindestange (4) kann somit in das Gehäuse (1a) bzw. in die Drehhülse (3) eingedreht werden, wobei die mit der Rasterung (1 j, 3j) zusammenwirkenden Rastelemente (4c) der Gewindestange nur eine Drehung der Gewindestange (4) relativ zum Gehäuse (1 a) bzw. relativ zur Drehhülse (3) in eine vorgegebene Richtung ermöglichen und in entgegengesetzter Richtung sperren, wie beispielhaft anhand der in den Fig. 3A und 3B gezeigten Ausführungsform erklärt.

Ebenso wie das in den Fig. 3A und 3B gezeigte Drehbegrenzungselement 6a oder 6b kann die in den Fig. 4A - 4C gezeigte Ausführungsform auch in Verbindung mit der in den Fig. 1 und 2 gezeigten Injektionsvorrichtung bzw. Dosiervorrichtung anstelle der dort beschriebenen Drehbegrenzungselemente 6a und 6b verwendet werden.

Das Sperrelement (5) kann verdrehgesichert auf der Gewindestange (4) gelagert werden, indem z.B. auf der Innenseite des Sperrelementes (5) eine Führungsrille oder Nut vorgesehen ist, in welche die Eingriffs- oder Rastelemente (4c) eingreifen können.

## Patentansprüche

1. Dosiervorrichtung zur Einstellung einer aus einer Injektionsvorrichtung (1) abzugebenden Dosis mit einem Einstellelement, insbesondere einer Einstellhülse (2), und einem Aufziehelement, insbesondere einer Drehhülse (3), das aus der Dasiervorrichtung (1) zur Vorbereitung einer Dosisabgabe herausgedreht werden kann, wobei auf dem Einstellelement (2) ein Anschlag (2b) und auf dem Aufziehelement (3) ein Gegenanschlag (3g) angebracht ist, so dass eine Drehbewegung des Aufziehelements (3) relativ zum Einstellelement (2) durch die Anschläge (2b, 3g) begrenzt werden kann, **dadurch gekennzeichnet, dass** das Einstellelement nur relativ zur injektionsvorrichtung (1) oder einem Gehäuse davon verdreht werden kann, wenn das Aufziehelement in einem vorgegebenen Lageverhältnis dazu ist.

2. Dosiervorrichtung nach Anspruch 1, wobei die aus der Injektionsvorrichtung (1) abzugebende Dosis durch ein Eindrehen des Aufziehelements (2) abgegeben werden kann.

3. Dosiervorrichtung nach einem der vorhergehenden Ansprüche, wobei die abzugebende Dosis proportional zu der axialen Aufziehweglänge des Aufziehelementes (2) ist.

4. Dosiervorrichtung nach einem der vorhergehenden Ansprüche, wobei der Anschlag (2b) und/oder Gegenanschlag (3g) als ein in axialer Richtung oder spiralförmig verlaufender Steg oder als vorstehende Nocke ausgebildet ist.

5. Dosiervorrichtung nach einem der Ansprüche 1 bis 3, wobei der Anschlag (2b) und/oder Gegenanschlag (3g) als ein zumindest zum Teil in Umfangsrichtung um das Einstellelement (2) oder das Aufziehelement (3) umlaufender Vorsprung oder Steg, insbesondere in Form eines Gewindes oder Gewindeabschnittes, ausgebildet ist.

6. Dosiervorrichtung nach einem der vorhergehenden Ansprüche, wobei der Anschlag (2b) und/oder der Gegenanschlag (3g) auf der Innenseite und/oder der Aussenseite des Einstellelements (2) oder des Aufziehelements (3) ausgebildet ist.

7. Dosiervorrichtung nach einem der vorhergehenden Ansprüche, wobei das Einstellelement (2) und das Aufziehelement (3) koaxial angeordnet und/oder zueinander verdrehbar sind.

8. Dosiervorrichtung zur Einstellung einer aus einer Injektionsvorrichtung abzugebenden Dosis mit einem Einstellelement, insbesondere einer Einstellhülse (2), und einem Aufziehelement, insbesondere einer Drehhülse (3), das aus der Dosiervorrichtung zur Vorbereitung einer Dosisabgabe herausgedreht werden kann, wobei mit dem Einstellelement (2) die abzugebende Dosis eingestellt werden kann und das Aufziehelement (3) und das Einstellelement (2) unabhängig voneinander bewegt werden kann, **dadurch gekennzeichnet, dass** das Einstellelement nur relativ zur Injektionsvorrichtung (1) oder einem Gehäuse davon verdreht werden kann, wenn das Aufziehelement in einem vorgegebenen Lageverhältnis dazu ist.

9. Dosiervorrichtung nach dem vorhergehenden Anspruch, wobei die Bewegung des Einstellelements (2) relativ zum Aufziehelement (3) durch einen Anschlag (2b) und/oder Gegenanschlag (3g) begrenzt werden kann.

10. Dosiervorrichtung nach einem der vorhergehenden Ansprüche, wobei das Einstellelements (2) relativ zur Injektionsvorrichtung (1) oder relativ zur Dosiervorrichtung durch ein Herausdrehen des Aufziehelements (3) festgestellt oder arretiert, insbesondere verriegelt werden kann.

11. Injektionsvorrichtung mit einer Dosiervorrichtung nach einem der vorhergehenden Ansprüche.

12. Injektionsvorrichtung nach dem vorhergehenden Anspruch, wobei das Einstellelement (2) relativ zur injektionsvorrichtung (1) mit einem Halteelement (2a), insbesondere einem radial vorgespannten Rastelement, festgestellt oder arretiert, insbesondere verriegelt werden kann.

13. Verfahren zum Einstellen einer Dosis, welche aus einer injektionsvorrichtung (1) abgegeben werden kann, wobei die abzugebende Dosis mit einem Einstellelement, insbesondere einer Einstellhülse (2), eingestellt werden kann und anschlisssend die injektionsvorrichtung (1) mit einem Aufziehelement, insbesondere einer Drehhülse (3), durch eine Drehbewegung aufgezogen werden kann, um in einem anschliessenden Schritt die eingestellte Dosis aus der injektionsvorrichtung (1) abzugeben, wobei der Einstellvorgang von dem Aufziehvorgang entkoppelt ist, wobei, während eines Aufzieh- und Abgabevorganges das Einstellelement relativ zu der injektionsvorrichtung oder einem Gehäuse davon nicht verdreht werden kann.

## Claims

1. Dosing device for regulating a dose to be delivered by an injection device (1), having a regulating element, in particular a regulating sleeve (2), and a drawing element, in particular a rotating sleeve (3) which can be rotated out of the dosing device (1) to prepare for a dose delivery, whereby a stop (2b) is placed on the regulating element (2) and a counter stop (3g) is placed on the drawing element (3) so that any rotational movement of the drawing element (3) relative to the regulating element (2) can be limited by the stops (2b,3g), **characterised in that** the regulating element can only be rotated relative to the injection device (1) or to a housing thereof when the drawing element is in a specified positional relationship thereto.

2. Dosing device according to claim 1, wherein the dose to be delivered by the injection device (1) can be delivered by screwing in the drawing element (2)¹.

3. Dosing device according to one of the preceding claims, wherein the dose to be delivered is proportional to the axial drawing distance of the drawing element (2)¹.

4. Dosing device according to one of the preceding claims, wherein the stop (2b) and/or counter stop (3g) is formed as a web running axially or in the form of a spiral or as a projecting cam.

5. Dosing device according to one of claims 1 to 3, wherein the stop (2b) and/or counter stop (3g) is formed as a projection or web running around the regulating element (2) or the drawing element (3) at least partially in the direction of the circumference, in particular in the form of a screw thread or screw thread section.

6. Dosing device according to one of the preceding claims, wherein the stop (2b) and/or the counter stop (3g) is formed on the inside and/or the outside of the regulating element (2) or of the drawing element (3).

7. Dosing device according to one of the preceding claims wherein the regulating element (2) and the drawing element (3) are arranged coaxially and/or can be twisted with respect to each other.

8. Dosing device for regulating a dose to be delivered by an injection device (1), having a regulating element, in particular a regulating sleeve (2), and a drawing element, in particular a rotating sleeve (3) which can be screwed out of the dosing device (1) to prepare for a dose delivery, wherein the dose to be delivered can be regulated using the regulating element (2) and the drawing element (3) and the regulating element (2) can be moved independently of each other, **characterised in that** the regulating element can only be twisted relative to the injection device (1) or to a housing thereof when the drawing element is in a specified positional relationship thereto.

9. Dosing device according to the preceding claim wherein the movement of the regulating element (2) relative to the drawing element (3) can be limited by a stop (2b) and/or a counter stop (3g).

10. Dosing device according to one of the preceding claims wherein the regulating element (2) can be fixed or arrested, in particular locked, relative to the injection device (1) or relative to the dosing device by unscrewing the drawing element (3).

11. Injection device with a dosing device according to one of the preceding claims.

12. Injection device according to the preceding claim, wherein the regulating element (2) can be fixed or arrested, in particular locked, relative to the injection device (1) with a holding element (2a), in particular a radially pre-tensioned detent element.

13. Method for regulating a dose which can be delivered from an injection device (1) wherein the dose to be delivered can be set using a regulating element, in particular a regulating sleeve (2) and subsequently the injection device (1) can be drawn up with a drawing device, in particular a drawing sleeve (3) by means of a rotational movement, in order in a subsequent step to deliver the regulated dose from the injection device (1), wherein the regulating process is decoupled from the drawing process, wherein, during a drawing and delivery process, the regulating element cannot be twisted relative to the injection device or a housing thereof.

## Revendications

1. Dispositif de dosage pour ajuster une dose à administrer à partir d'un dispositif d'injection (1) avec
un élément d'ajustement, en particulier, une douille d'ajustement (2), et
un élément d'amorçage, en particulier une douille rotative (3) qui peut être tournée à partir du dispositif de dosage (1) pour préparer l'administration d'une dose,
dans lequel il est prévu une butée (2b) sur l'élément d'ajustement (2) et une contrebutée (3g) sur l'élément d'amorçage (3), de sorte qu'un mouvement rotatif de l'élément d'amorçage (3) par rapport à l'élément d'ajustement (2) puisse être limité par les butées (2b, 3g),
**caractérisé en ce que** l'élément d'ajustement ne peut être tourné que relativement au dispositif d'injection (1) ou à un boîtier de celui-ci, lorsque l'élément d'amorçage est dans un rapport de position prédéterminé par rapport à celui-ci.

2. Dispositif de dosage selon la revendication 1, dans lequel la dose à administrer à partir du dispositif d'injection (1) peut être administrée par un tour de l'élément d'amorçage (3).

3. Dispositif de dosage selon l'une des revendications précédentes, dans lequel la dose à administrer est proportionnelle à la longueur de course de vissage axial de l'élément d'amorçage (3).

4. Dispositif de dosage selon l'une des revendications précédentes, dans lequel la butée (2b) et/ou la contrebutée (3g) sont conçues comme un pas évoluant en direction axiale ou sous forme de spirale ou comme une came proéminente.

5. Dispositif de dosage selon l'une des revendications 1 à 3, dans lequel la butée (2b) et/ou la contrebutée (3g) est conçue comme une proéminence ou un pas entourant au moins en partie dans la direction périphérique, l'élément d'ajustement (2) ou l'élément d'amorçage (3), en particulier sous la forme d'un filetage ou d'un tronçon de filetage.

6. Dispositif de dosage selon l'une des revendications précédentes, dans lequel la butée (2b) et/ou la contrebutée (3g) est formée sur le côté interne et/ou le côté externe de l'élément d'ajustement (2) ou de l'élément d'amorçage (3).

7. Dispositif de dosage selon l'une des revendications précédentes, dans lequel l'élément d'ajustement (2) et l'élément d'amorçage (3) sont disposés de façon coaxiale et/ou de façon à pouvoir tourner l'un par rapport à l'autre.

8. Dispositif de dosage pour l'ajustement d'une dose à administrer à partir d'un dispositif d'injection avec
un élément d'ajustement, en particulier, une douille d'ajustement (2), et
un élément d'amorçage, en particulier une douille rotative (3) qui peut être tournée à partir du dispositif de dosage pour préparer l'administration d'une dose,
dans lequel la dose à administrer peut être ajustée avec l'élément d'ajustement (2), et l'élément d'amorçage (3) et l'élément d'ajustement (2) peuvent être mobiles indépendamment l'un de l'autre,
**caractérisé en ce que** l'élément d'ajustement ne peut être tourné que relativement au dispositif d'injection (1) ou à un boîtier de celui-ci, lorsque l'élément d'amorçage est dans un rapport de position prédéterminé par rapport à celui-ci.

9. Dispositif de dosage selon la revendication précédente, dans lequel le mouvement de l'élément d'ajustement (2) par rapport à l'élément d'amorçage (3) peut être limité par une butée (2b) et/ou une contrebutée (3g).

10. Dispositif de dosage selon l'une des revendications précédentes, dans lequel l'élément d'ajustement (2) peut être fixé ou arrêté, en particulier, verrouillé par rapport au dispositif d'injection (1) ou par rapport au dispositif de dosage par un dévissage de l'élément d'amorçage (3).

11. Dispositif d'injection avec un dispositif de dosage selon l'une des revendications précédentes.

12. Dispositif d'injection selon la revendication précédente, dans lequel l'élément d'ajustement (2) peut être fixé ou arrêté, en particulier verrouillé par rapport au dispositif d'injection (1) avec un élément d'arrêt (2a), en particulier, un élément d'encliquetage précontraint au niveau radial.

13. Procédé d'ajustement d'une dose qui peut être administrée à partir d'un dispositif d'injection (1), dans lequel la dose à administrer peut être ajustée avec un élément d'ajustement, en particulier, une douille d'ajustement (2) et ensuite, le dispositif d'injection (1) peut être amorcé avec un élément d'amorçage, en particulier une douille rotative (3) par un mouvement rotatif, pour distribuer, dans une étape suivante, la dose ajustée à partir du dispositif d'injection (1), le processus d'ajustement étant découplé du processus d'amorçage, dans lequel, pendant un processus d'amorçage et d'administration, l'élément d'ajustement ne peut pas être tourné par rapport au dispositif d'injection ou à un boîtier de celui-ci.
